# EUROPÄISCHE PATENTSCHRIFT

(11) **EP 2 879 147 B1**
(45) Veröffentlichungstag und Bekanntmachung des Hinweises auf die Patenterteilung: **23.08.2017**
(21) Anmeldenummer: 14193127.9
(22) Anmeldetag: 14.11.2014
(51) Int. Cl.: H01H 13/06, H01H 9/06

(54) **Tastschalterbaugruppe für ein Handstück zur Durchführung medizinischer Eingriffe**
Pushbutton switch for a handpiece for performing medical procedures
Bloc de bouton-poussoir pour une pièce à main destinée à l'exécution d'opérations médicales

(30) Priorität: 27.11.2013 DE 102013224213
(43) Veröffentlichungstag der Anmeldung: 03.06.2015
(73) Patentinhaber: Karl Storz GmbH & Co. KG, 78532 Tuttlingen (DE)
(72) Erfinder: Spycher, Raphael, 8264 Eschenz (CH); Simmen, Markus, 8603 Schwerzenbach (CH)
(74) Vertreter: Ruttensperger, Bernhard

(56) Entgegenhaltungen:
- EP-A1- 2 319 419
- CN-U- 203 026 410
- US-A1- 2001 017 255
- US-A1- 2013 292 235
- US-B2- 8 594 597

## Beschreibung

Die vorliegende Erfindung betrifft eine Tastschalterbaugruppe für ein Handstück gemäß dem Oberbegriff des Anspruchs 1, welches bei der Durchführung medizinischer Eingriffe, insbesondere chirurgischer Eingriffe, eingesetzt werden kann.

Ein Handstück zur Durchführung medizinischer Eingriffe ist aus der US 6,500,169 B1 bekannt. Dieses Handstück enthält einen Antrieb für ein an dem Handstück anzubringendes chirurgisches Werkzeug. Ein derartiges chirurgisches Werkzeug kann ein so genannter Orthopädieshaver sein, mit welchem in Bohr- bzw. Fräsvorgängen im Inneren eines Körpers Material, beispielsweise Gewebe- oder Knochenmaterial, abgetragen werden kann. An diesem bekannten Handstück ist eine Tastschalterbaugruppe mit drei in Reihe angeordneten, manuell zu betätigenden Beaufschlagungsbereichen vorgesehen, welche bei manueller Betätigung auf Schalterelemente einwirken und somit eine Aktivierung bzw. Ansteuerung des im Handstück vorgesehenen Antriebs ermöglichen. Die drei in einer Reihe angeordneten Beaufschlagungsbereiche sind an einem plattenartigen, flexiblen Tasterelement ausgebildet. Das Tasterelement ist eine Aussparung am Handstück überdeckend angeordnet. Ein das Tasterelement übergreifend positioniertes, plattenartiges Abdeckelement wird durch eine Mehrzahl von Befestigungsschrauben am Handstück festgelegt und presst dabei einen Außenrandbereich des Tasterelements gegen eine Widerlagerfläche des Handstücks, um einen fluiddichten Abschluss herzustellen.

Aus der EP 2 319 419 A1 ist eine Tastschalterbaugruppe bekannt. Diese bekannte Tastschalterbaugruppe umfasst einen plattenartigen Träger mit zwei darin vorgesehenen Öffnungen zur Aufnahme jeweils eines Tasterelements. Durch Druckbeaufschlagung eines jeweiligen Tasterelements wird dieses verformt, wodurch ein am Tasterelement vorgesehener Stift in Richtung auf einen jeweils zugeordneten elektrischen Schalter zu verschoben wird, der in einem Gehäuse eines chirurgischen Handstücks vorgesehen ist.

Die US 2001/0017255 A1 offenbart einen elektrischen Rasierer mit einer Tastschalteranordnung. Durch die an einem Deckelelement vorgesehene Tastschalteranordnung kann ein im Inneren des Rasierergriffs vorgesehener elektrischer Schalter betätigt werden.

Eine Tastschalterbaugruppe gemäß dem Oberbegriff des Anspruchs 1 ist aus der US 2013/292235 A1 bekannt.

Es ist die Aufgabe der vorliegenden Erfindung, eine Tastschalterbaugruppe für ein Handstück zur Durchführung medizinischer Eingriffe vorzusehen, welches bei einfachem, zuverlässig wirkendem Aufbau die Erzeugung eines fluiddichten Abschlusses bezüglich eines die Tastschalterbaugruppe aufnehmenden Handstücks ermöglicht.

Erfindungsgemäß wird diese Aufgabe gelöst durch eine Tastschalterbaugruppe für ein Handstück zur Durchführung medizinischer Eingriffe gemäß Anspruch 1. Diese umfasst, inter alia:
- einen einen Trägerinnenraum umgebenden ringartigen Träger,
- an dem Träger ein zur Durchführung von Schaltvorgängen zu betätigendes Tasterelement, wobei das Tasterelement an dem Träger den Trägerinnenraum fluiddicht abschließend festgelegt ist,
- an einer Außenumfangsseite des ringartigen Trägers eine den ringartigen Träger umgebende Dichtungsanordnung.

Beim erfindungsgemäßen Aufbau bilden der ringartige Träger und das daran festgelegte Tasterelement eine Einheit, welche für sich bereits dafür sorgt, dass im Angrenzungsbereich des Tasterelements an den ringartigen Träger durch den dort erzeugten fluiddichten Abschluss das Eindringen von Fluid nicht möglich ist. Der fluiddichte Anschluss des Trägers selbst an ein diesen aufnehmendes Handstück erfolgt durch die an der Außenumfangsseite des Trägers vorgesehene Dichtungsanordnung. Somit ist ein einfach zu realisierender Aufbau der Tastschalterbaugruppe bereitgestellt, bei welchem insbesondere der fluiddichte Anschluss an ein diese aufnehmendes Handstück nicht durch ein flächenartig ausgestaltetes und durch ein zusätzliches Bauteil gepresstes Dichtelement, sondern durch eine an der Außenumfangsseite des ringartigen Trägers und somit im Wesentlichen radial wirkende Dichtungsanordnung erzeugt ist. Die Bereitstellung eines derartigen fluiddichten Abschlusses unter Vermeidung des Entstehens von kleinen Strukturen, wie sie insbesondere im Bereich von Befestigungsschrauben auftreten können, ist vor allem bei der Durchführung von Reinigungsvorgängen derartiger insbesondere im Bereich chirurgischer Eingriffe einzusetzender Geräte von wesentlicher Bedeutung.

Um durch die Tastschalterbaugruppe ein beispielsweise elektrisch zu erregendes Gerät, beispielsweise einen Elektroantriebsmotor, ansteuern bzw. aktivieren oder deaktivieren zu können, ist eine Schalteranordnung mit wenigstens einem durch das Tasterelement beaufschlagbaren Schalterelement vorgesehen. Dabei umfasst die Schalteranordnung eine im Wesentlichen im Trägerinnenraum angeordnete und wenigstens ein Schalterelement tragende Schalterplatte, also beispielsweise Leiterplatte.

Um den fluiddichten Anschluss des Tasterelements an den ringartigen Träger in besonders einfacher und zuverlässiger Weise erreichen zu können, wird vorgeschlagen, dass das Tasterelement im Wesentlichen plattenartig ausgebildet ist und zur fluiddichten Festlegung am Träger einen eine Stirnseite des Trägers wenigstens bereichsweise übergreifenden Befestigungsflansch aufweist oder/und eine in den Trägerinnenraum eingreifende und an einer Innenumfangsseite des Trägers anliegende Befestigungswandung aufweist.

Die Festlegung des Tasterelements an dem Träger erfolgt vorteilhafterweise durch Materialschluss, vorzugsweise Anvulkanisieren. Durch das Anvulkanisieren wird einerseits eine fluiddichte Anbindung gewährleistet, andererseits ist eine derartige Anbindung mechanisch stark belastbar.

Ein besonders einfacher, gleichwohl jedoch eine optimale Abdichtung der Tastschalterbaugruppe bezüglich eines diese aufnehmenden Handstücks unterstützender Aufbau kann dadurch erreicht werden, dass die Dichtungsanordnung an der Außenumfangsseite des Trägers in wenigstens einer Dichtelementaufnahmenut wenigstens ein O-ringartiges Dichtelement umfasst.

Die Dichtwirkung der Dichtungsanordnung kann besonders effizient und baulich einfach erreicht werden, wenn der Träger eine im Wesentlichen runde, vorzugsweise kreisrunde, Außenumfangskontur aufweist. Durch die im Wesentlichen runde Ausgestaltung wird das Entstehen von Eck- oder Kantenbereichen, an welchen die Dichtwirkung geschwächt sein könnte, vermieden. Es ist darauf hinzuweisen, dass eine kreisrunde Ausgestaltung aufgrund der Minimierung des abzudichtenden Umfangs bei vorgegebener Querschnittsfläche besonders vorteilhaft ist. Grundsätzlich könnte jedoch auch eine elliptische, ovale oder in ähnlicher Weise geformte Außenumfangskontur vorgesehen sein.

Zum Gewährleisten einer definierten Wechselwirkung zwischen dem Tasterelement und dem wenigstens einen an der Schalterplatte vorgesehenen Schalterelement wird vorgeschlagen, dass am Tasterelement oder/und am Träger eine Positionierungsformation vorgesehen ist und dass an der Schalterplatte eine mit der Positionierungsformation zum Vorgeben einer vorbestimmten Positionierung für die Schalterplatte im Trägerinnenraum zusammenwirkende Gegen-Positionierungsformation vorgesehen ist.

Diese Positionierungswechselwirkung kann in einfacher Weise dadurch erreicht werden, dass eine Formation von Positionierungsformation und Gegen-Positionierungsformation, vorzugsweise die Positionierungsformation, wenigstens einen Positioniervorsprung umfasst und die andere Formation von Positionierungsformation und Gegen-Positionierungsformation, vorzugsweise die Gegen-Positionierungsformation, wenigstens eine wenigstens einen Positioniervorsprung aufnehmende Positionieraussparung umfasst.

Bei Durchführung von Schaltvorgängen wird durch beispielsweise manuelle Betätigung auf das im Allgemeinen flexibel ausgebildete Tasterelement eingewirkt und dieses verformt. Um dabei eine definierte Wechselwirkung zwischen dem Tasterelement und der Schalteranordnung zu gewährleisten, weist das Tasterelement in Zuordnung zu wenigstens einem Schalterelement, vorzugsweise in Zuordnung zu jedem Schalterelement, einen Beaufschlagungsbereich auf.

Eine einerseits das Vermeiden von Betätigungsfehlern unterstützende und andererseits eine definierte Einwirkung auf ein vorbestimmtes Schalterelement gewährleistende Ausgestaltung kann vorsehen, dass wenigstens ein Beaufschlagungsbereich eine an einer vom Trägerinnenraum weg orientierten Außenseite des Tasterelements vorgesehene erste Beaufschlagungsformation, vorzugsweise erster Beaufschlagungsvorsprung umfasst.

Wenigstens ein Beaufschlagungsbereich umfasst an einer zum Trägerinnenraum hin orientierten Innenseite des Tasterelements einen zweiten Beaufschlagungsvorsprung.

Bei der manuellen Betätigung der Tastschalterbaugruppe ist es von besonderer Bedeutung, dass für eine Bedienperson, also beispielsweise einen Arzt, während der Durchführung eines chirurgischen Eingriffs erkennbar wird, dass bei Einwirkung auf das Tasterelement tatsächlich auch ein Schaltvorgang erfolgt. Hierfür kann in Zuordnung zu wenigstens einem Schalterelement, vorzugsweise in Zuordnung zu jedem Schalterelement, ein der Beaufschlagungswirkung des Tasterelements entgegenwirkendes, vorzugsweise monostabiles Beaufschlagungswiderstandskraftelement vorgesehen sein. Das Beaufschlagungswiderstandskraftelement erzeugt bei Belastung des Tasterelements eine Widerstandskraft, welche der Bedienperson signalisiert, dass das Tasterelement in ausreichendem Ausmaß betätigt wurde.

Um eine definierte Zuordnung eines derartigen Beaufschlagungswiderstandskraftelements zu einem Schalterelement der Schalteranordnung zu gewährleisten, wird vorgeschlagen, dass wenigstens ein Beaufschlagungswiderstandskraftelement durch eine Fixierlage, vorzugsweise Klebefolie, bezüglich der Schalteranordnung fixiert ist.

Die Erfindung betrifft ferner ein Handstück zur Durchführung medizinischer, insbesondere chirurgischer Eingriffe, umfassend wenigstens eine erfindungsgemäß aufgebaute Tastschaltergruppe, wobei in dem Handstück in Zuordnung zu der wenigstens einen Tastschalterbaugruppe eine diese aufnehmende Tastschalteraufnahmeaussparung vorgesehen ist.

Der fluiddichte Anschluss der Tastschalterbaugruppe an das Handstück kann vorteilhafterweise dadurch erreicht werden, dass die Dichtungsanordnung an einer die Tastschalteraufnahmeaussparung umgebenden Innenumfangsfläche fluiddicht anliegt. Diese fluiddichte und im Allgemeinen unter Vorspannung erfolgende Anlage kann gleichzeitig auch dazu dienen, die gesamte Tastschalterbaugruppe an dem Handstück fixiert zu halten. Des Weiteren kann vorgesehen sein, dass bei der Tastschalteraufnahmeaussparung wenigstens eine Haltestufe vorgesehen ist. Diese Haltestufe kann einerseits dazu dienen, einen Einschiebeanschlag für die Tastschalterbaugruppe beim Einschieben derselben in die Tastschalteraufnahmeaussparung bereitzustellen und somit eine definierte Positionierung der Tastschalterbaugruppe in der Tastschalteraufnahmeaussparung zu gewährleisten. Des Weiteren kann diese wenigstens eine Haltestufe dazu dienen, ein Herausbewegen der im Trägerinnenraum positionierten Schalteranordnung aus dem Trägerinnenraum zu verhindern. Es sind dann keine weiteren, eine wesentliche Befestigungskraft für die Schalteranordnung am Träger gewährleistende baulichen Maßnahmen erforderlich.

Die vorliegende Erfindung wird nachfolgend mit Bezug auf die beiliegenden Figuren detailliert beschrieben. Es zeigt:
- Fig. 1: eine perspektivische Ansicht eines Handstücks zur Durchführung medizinischer Eingriffe;
- Fig. 2: eine vergrößerte Detailansicht des Handstücks der Fig. 1 mit in Explosionsansicht über einer Tastschalteraufnahmeaussparung gezeigter Tastschalterbaugruppe;
- Fig. 3: die Tastschalterbaugruppe in vergrößerter Explosionsdarstellung;

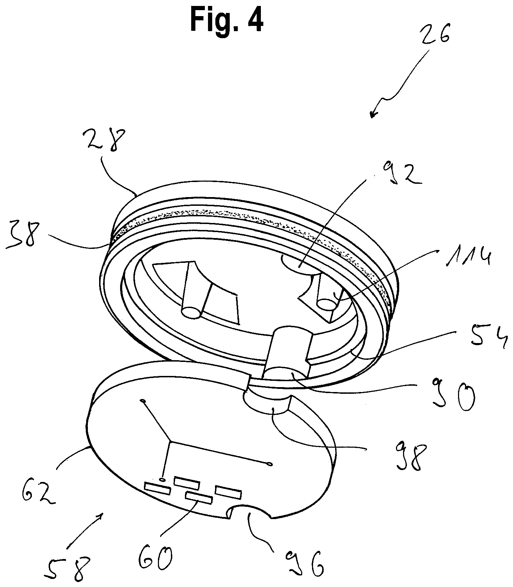

- Fig. 4: in perspektivischer Explosionsdarstellung einen mit einer Dichtungsanordnung und einem Tasterelement versehenen ringartigen Träger und eine in einem Trägerinnenraum zu positionierende Schalteranordnung der Tastschalterbaugruppe;
- Fig. 5: eine der Fig. 4 entsprechende Schnittdarstellung;

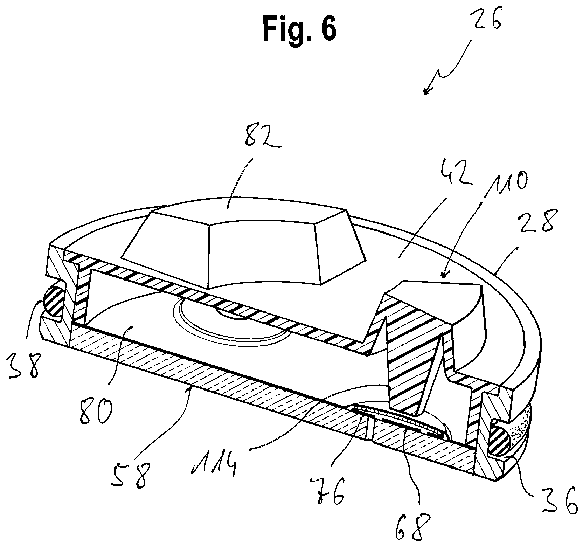

- Fig. 6: eine perspektivische Schnittdarstellung der Tatschalterbaugruppe im zusammengefügten Zustand;

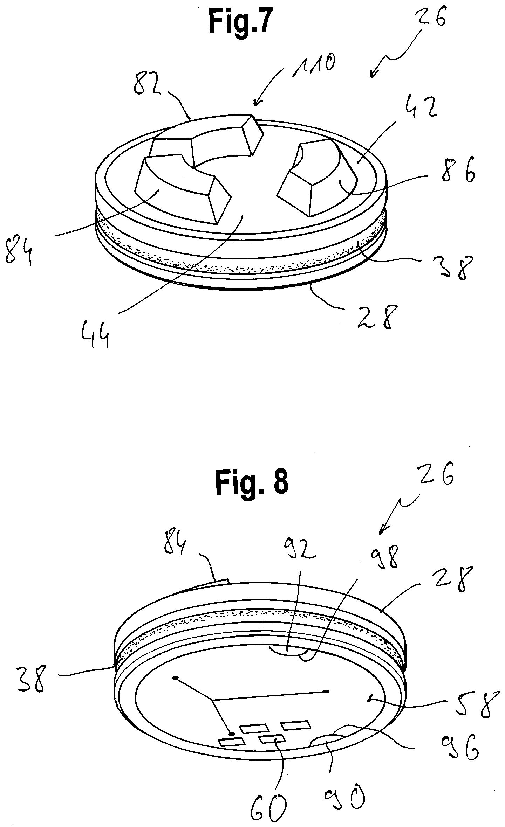

- Fig. 7: eine perspektivische Ansicht der Tastschalterbaugruppe von oben;
- Fig. 8: eine perspektivische Ansicht der Tastschalterbaugruppe von unten.

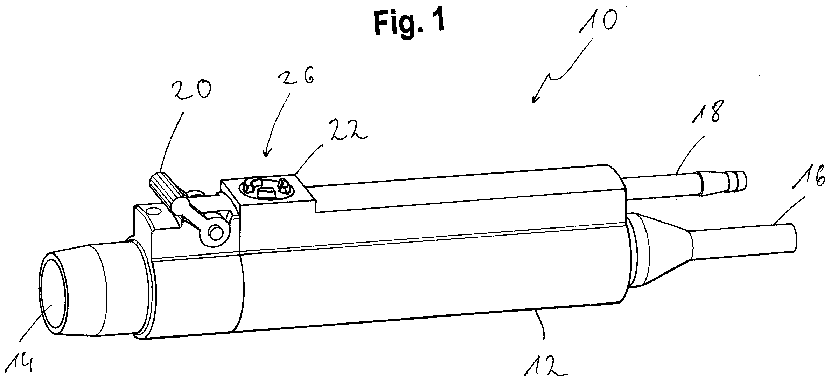

In Fig. 1 ist ein zur Durchführung medizinischer Eingriffe einsetzbares Handstück allgemein mit 10 bezeichnet. Das Handstück 10 enthält in einem Handstückgehäuse 12 einen beispielsweise elektromotorischen Antrieb für ein in Fig. 1 nicht dargestelltes, am Handstück 10 festzulegendes chirurgisches Werkzeug. Dieses kann in einen in Fig. 1 links dargestellten Endbereich, insbesondere eine dort vorgesehene Öffnung 14, eingesetzt werden und mit dem im Handstückgehäuse 12 aufgenommenen Antrieb verkoppelt werden, so dass dieses chirurgische Werkzeug beispielsweise zur Drehung um seine Längsachse angetrieben werden kann. Beispielsweise kann dieses chirurgische Werkzeug als Orthopädieshaver ausgebildet sein, mit welchem in einem Körper, beispielsweise menschlichem Körper, Gewebe oder Knochenmaterial abgetragen werden kann. Zur Versorgung mit elektrischer Energie ist am Handstück 10 ein Anschlusskabel 16 vorgesehen. Ferner ist am Handstück 10 ein Anschlussstutzen 18 vorgesehen. An diesen Anschlussstutzen 18 kann eine Saugleitung angeschlossen werden, um während eines operativen Eingriffs durch das chirurgische Werkzeug abgetragenes Material durch einen im Inneren dieses Werkzeugs gebildeten Hohlraum hindurch in das Handstück 10 und durch den Anschlussstutzen 18 hindurch abzusaugen. Auch in den Bereich, in welchem ein derartiger operativer Eingriff vorgenommen wird, eingeleitete Spülflüssigkeit kann auf diese Art und Weise durch das Handstück 10 hindurch abgesaugt werden. Zur Aktivierung dieser Absaugfunktion ist am Handstück 10 ein Betätigungshebel 20 vorgesehen, der durch manuelle Betätigung beaufschlagt und dabei verschwenkt werden kann, um ein im Handstück 10 vorgesehenes Ventil zur vorzugsweise stufenlosen Freigabe eines Strömungswegs durch das Handstück 10 hindurch zu betätigen.

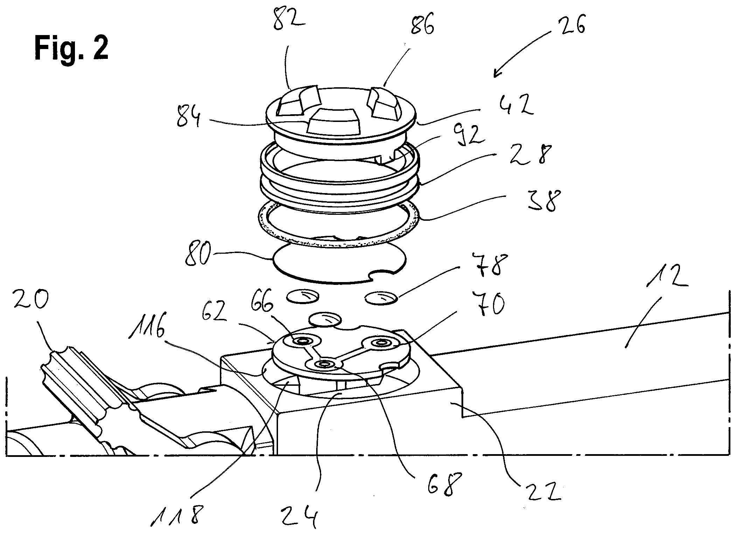

Wie dies auch in Fig. 2 erkennbar ist, ist in einem beispielsweise erhöhten Bereich 22 des Handstückgehäuses 12 eine Tastschalteraufnahmeaussparung 24 ausgebildet. In diese Tastschalteraufnahmeaussparung 24 ist eine nachfolgend detailliert beschriebene Tastschalterbaugruppe 26 derart eingesetzt, dass durch manuelle Beaufschlagung der im Handstück 10 vorgesehene Antrieb, also beispielsweise ein elektrisch erregbarer Antriebsmotor, angesteuert bzw. aktiviert oder deaktiviert werden kann.

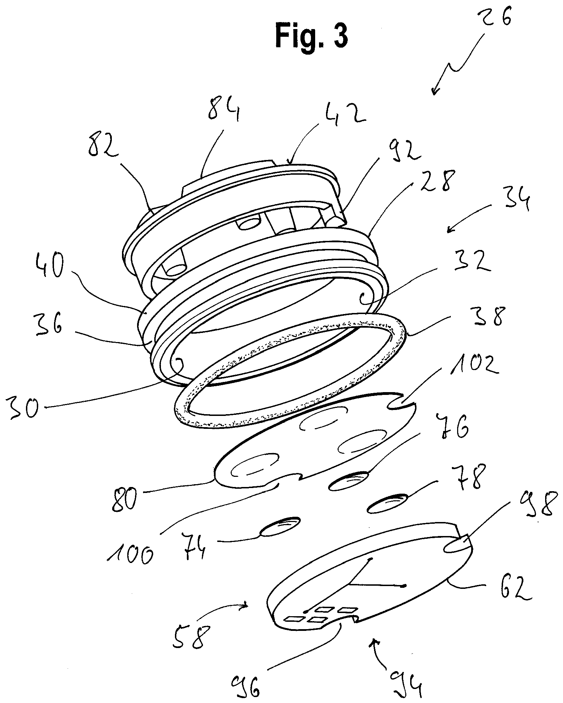

Die auch in Fig. 3 in Explosionsansicht dargestellte Tastschalterbaugruppe 26 weist als zentrales Bauteil einen vorteilhafterweise aus Metallmaterial, beispielsweise Edelstahl, aufgebauten, ringartigen Träger 28 auf. Der ringartige Träger 28 ist an seiner Innenumfangsseite 30 beispielsweise mit einer im Wesentlichen planen, also zylindrischen Innenumfangsfläche 32 ausgebildet. An seiner Außenumfangsseite 34 weist der ringartige Träger 28 eine über den gesamten Umfang vorzugsweise mit im Wesentlichen konstanter Breite oder/und Tiefe sich erstreckende und bezüglich einer Längsmittenachse des ringartigen Trägers 28 nach radial außen offenen Nut 36 auf. In dieser Nut 36 ist im Zusammenbau der Tastschalterbaugruppe 26 ein O-ringartiges Dichtelement 38 angeordnet, und zwar derart, dass es über eine Außenumfangsfläche 40 des ringartigen Trägers 28 im unbelasteten Zustand hervorsteht.

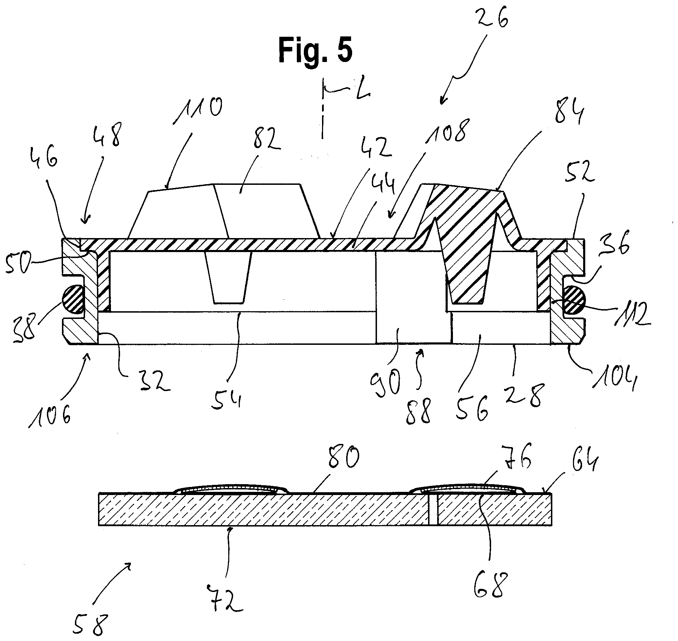

Zur manuellen Betätigung, beispielsweise durch einen einen chirurgischen Eingriff vornehmenden Arzt, weist die Tastschalterbaugruppe 26 ein beispielsweise im Wesentlichen plattenartig ausgebildetes und aus Elastomermaterial, also flexibel, aufgebautes Tasterelement 42 auf. In der Schnittdarstellung der Fig. 5 ist zu erkennen, dass das Tasterelement 42 einen plattenartigen Körper 44 aufweist, der in seinem radial äußeren Bereich, radial bezogen auf eine Längsmittenachse L des ringartigen Trägers 28, einen in einer entsprechenden Einsenkung einer Stirnseite 48 des ringartigen Trägers 28 aufgenommenen Befestigungsflansch 50 bereitstellt. Somit liegt der Körper 44 des Tasterelements 42 in diesem Bereich im Wesentlichen bündig mit einer Stirnfläche 52 des Trägers 28.

Vom plattenartigen Körper 44 erstreckt sich eine vorzugsweise im Wesentlichen zylindrisch ausgestaltete Befestigungswandung 54 derart, dass sie bei in der Einsenkung 46 aufgenommenem Befestigungsflansch 50 an der Innenumfangsfläche 32 des Trägers 28 anliegt. Im Bereich der Anlage des Befestigungsflansches 50 oder/und der Befestigungswandung 54 des Tasterelements 42 am ringartigen Träger 28 sind diese beiden Bauteile vorzugsweise materialschlüssig fest und damit auch fluiddicht miteinander verbunden. Dieser Materialschluss kann in besonders vorteilhafter Weise durch Anvulkanisieren des beispielsweise aus Elastomermaterial aufgebauten Tasterelements 42 erfolgen. Grundsätzlich ist auch der Einsatz von Klebstoff oder dergleichen möglich. Der ringartige Träger 28 und das Tasterelement 42 bilden somit eine miteinander fest verbundene Baueinheit, welche in Richtung zur Stirnseite 48 des ringartigen Trägers einen von diesem umschlossenen Trägerinnenraum 56 fluiddicht abschließt.

Die Tastschalterbaugruppe 26 umfasst ferner eine allgemein mit 58 bezeichnete Schalteranordnung. Diese Schalteranordnung 58 weist eine beispielsweise nach Art einer Leiterplatte aufgebaute, z. B. im Wesentlichen zylindrische und eine Mehrzahl von Leiterbahnen bzw. auch Kontakten 60 tragende Schalterplatte 62 auf. An einer dem Trägerinnenraum 56 zugewandt zu positionierenden Innenseite 64 der Schalteranordnung 58 trägt die Schalterplatte 62 im dargestellten Beispiel drei Schalterelemente 66, 68, 70. Jedes dieser Schalterelemente 66, 68, 70, die, wie die Fig. 2 dies zeigt, in einer dreieckartigen bzw. einer ringartigen Konfiguration an der Schalterplatte 62 positioniert sein können, kann dazu dienen, durch Schließen eines elektrischen Kontakts beispielsweise einen elektrisch erregbaren Antriebsmotor in definierter Art und Weise anzusteuern. Dabei kann die elektrische Verbindung zwischen der Schalteranordnung 58 und weiteren im Handstück 10 vorgesehenen Leitungselementen über die Kontakte 60, z. B. Lötkontakte, an einer vom Trägerinennraum 56 abgewandt zu positionierenden Außenseite 72 der Schalteranordnung 58 erfolgen.

In Zuordnung zu jedem der drei Schalterelemente 66, 68, 70 kann ein Beaufschlagungswiderstandskraftelement 74, 76, 78 vorgesehen sein. Diese beispielsweise gewölbt, plättchenartig ausgebildeten und vorteilhafterweise monostabil wirksamen Beaufschlagungswiderstandskraftelemente werden über den Schalterelementen 66, 68, 70 positioniert und durch eine Klebefolie 80, z. B. Polyimid-Folie, auf der Schalterplatte 62 gehalten. Dabei kann eine Außenumfangskontur der Klebefolie 80 im Wesentlichen der Außenumfangskontur der Schalterplatte 62 entsprechen.

In Zuordnung zu jedem der drei Schalterelemente 66, 68, 70 ist am Tasterelement 42 ein Beaufschlagungsbereich 82, 84, 86 vorgesehen. Die Beaufschlagungsbereiche 82, 84, 86 sind im Wesentlichen zueinander gleich gestaltet und am Tasterelement 42 so positioniert, dass sie über den jeweils zugeordneten Schalterelementen, 66, 68, 70 bzw. den Beaufschlagungswiderstandskraftelementen 74, 76, 78 liegen. Um diese definierte Relativpositionierung zu gewährleisten, ist am Tasterelement 42 eine Positionierungsformation 88 vorgesehen. Diese umfasst an zwei Umfangsbereichen Positioniervorsprünge 90, 92, welche sich im Bereich der Befestigungswandung 54 über diese axial hinaus und beispielsweise auch radial nach innen erstrecken. In der Schalteranordnung 58 bzw. der Schalterplatte 62 derselben und auch der Klebefolie 80 sind zum Bereitstellen einer Gegen-Positionierformation 94 Positonieraussparungen 96, 98 bzw. 100, 102 vorgesehen. Im Zusammenbauzustand greifen die Positioniervorsprünge 90, 92 in die Positionieraussparungen 96, 98 der Schalterplatte 62 ein und sorgen dafür, dass bei im Trägerinnenraum 56 positionierter Schalteranordnung 58 diese eine definierte Positionierung bezüglich des Tasterelements 42 aufweist und sich nicht verdrehen kann. Um auch eine definierte axiale Positionierung bezogen auf die Längsmittenachse L des ringartigen Trägers 28 zu gewährleisten, kann beispielsweise die Befestigungswandung 54 des Tasterelements 42 sich so weit in den Trägerinnenraum 56 hinein erstrecken, dass dadurch ein Einschubanschlag für die Schalteranordnung 58 gebildet ist, so dass im zusammengesetzten Zustand die Rückseite 72 der Schalteranordnung 58 im Wesentlichen bündig mit einer Stirnfläche 104 an einer Stirnseite 106 des ringartigen Trägers 28 liegen kann.

Die vorangehend bereits angesprochenen Beaufschlagungsbereiche 82, 84, 86 des Schalterelements 42 weisen vom plattenartigen Körper 44 des Tasterelements 42 sich an einer Außenseite 108 desselben in Richtung vom Trägerinnenraum 56 weg erstreckende, kuppelartige erste Beaufschlagungsvorsprünge 110 auf. Diese sind für einen das Handstück 10 benutzenden Arzt mit dem zur Betätigung verwendeten Finger, beispielsweise dem Daumen, erfüllbar, so dass, je nach dem, auf welches der der Schalterelemente 66, 68, 70 eingewirkt werden soll, der Beaufschlagungsvorsprung 110 des zugeordneten Beaufschlagungsbereichs 82, 84, 86 beaufschlagt, also drückend belastet wird.

Jeder Beaufschlagungsbereich 82, 84, 86 weist des weiteren im Bereich eines jeweiligen ersten Beaufschlagungsvorsprungs 110 einen an einer Innenseite 112 des plattenartigen Körpers 44 des Tasterelements 42 sich in Richtung in den Trägerinnenraum 56 hinein erstreckenden zweiten Beaufschlagungsvorsprung 114 auf. Jeder dieser zweiten Beaufschlagungsvorsprünge 114 ist so dimensioniert, dass er im nicht belasteten Zustand beispielsweise mit geringem Abstand über einem jeweils zugeordneten Schalterelement 66, 68, 70 bzw. Beaufschlagungswiderstandskraftelement 74, 76, 78 liegt. Wird auf den ersten Beaufschlagungsvorsprung 110 eines Beaufschlagungsbereichs 82, 84, 86 gedrückt und dieser somit in Richtung zum Trägerinnenraum 56 verschoben, so verschiebt sich entsprechend auch der zugeordnete zweite Beaufschlagungsvorsprung 114 in Richtung auf das zugeordnete Schalterelement 66, 86 oder 70 zu. Im Verlaufe dieser Verschiebebewegung kommt dieser zweite Beaufschlagungsvorsprung 114 in Kontakt mit der Klebefolie 80 und belastet somit das darunter liegende Beaufschlagungswiderstandskraftelement 74, 76 oder 78. Dieses monostabile Bauteil erzeugt zunächst eine deutlich spürbare Widerstandskraft, gibt aber bereits bei geringer Belastung nach, so dass im Zuge der fortgesetzten Bewegung des zweiten Beaufschlagungsvorsprungs 114 dieser auch das Schaltelement 66, 68 oder 70 belastet und beispielsweise einen elektrischen Kontakt dieses Schalterelements schließt. Dabei signalisiert der Übergang eines jeweiligen Beaufschlagungswiderstandskraftelements 74, 76, 78 in seinen instabilen Zustand einer Bedienperson, dass der jeweilige Beaufschlagungsbereich 82, 84 oder 86 in derartigem Ausmaß belastet wurde, dass auch das zugeordnete Schalterelement 66, 68, 70 betätigt wurde.

Wird die Beaufschlagungswirkung gemindert bzw. aufgehoben, so kehrt das Tasterelement 42 aufgrund seiner Elastizität in seine Ausgangsform zurück. Dabei gibt der zuvor jeweils wirksame zweite Beaufschlagungsvorsprung 114 das mit diesem zusammenwirkende Schalterelement 66, 68 bzw. 70 frei, so dass beispielsweise der zuvor hergestellte elektrische Kontakt wieder unterbrochen wird und auch das zugeordnete Beaufschlagungswiderstandskraftelement in seinen stabilen Zustand zurückkehrt.

Es ist hier darauf hinzuweisen, dass in dem in den Figuren dargestellten Ausgestaltungsbeispiel die Anzahl und auch die Positionierung der Schalterelemente 66, 68, 78 und auch der damit zusammenwirkenden Bauteile bzw. Abschnitte derselben nur beispielhaft gewählt ist. Es könnten selbstverständlich auch mehr oder weniger Schalterelemente vorgesehen sein. Die Auswahl der Anzahl der Schalterelemente kann abhängig davon gewählt werden, welche bzw. wie viele Funktionen durch eine derartige Tastschalterbaugruppe 26 zu bedienen sind. Diese Funktionen können beispielsweise das Ein- und Ausschalten eines Elektromotors für ein chirurgisches Werkzeug, die Vorgabe der Drehrichtung, die Vorgabe der Drehzahl und den Betrieb, also beispielsweise Bohren oder Fräsen, umfassen. Auch kann die Tastschalterbaugruppe durch Wechselwirkung des Tasterelements 42 mit der Schalteranordnung 58 zur Realisierung einer Dimmer-Funktionalität genutzt werden, bei welcher beispielsweise gesteuert durch die Zeitdauer der Beaufschlagung des Tasterelements 42 eine stufenlose Variation der Drehzahl eines Elektromotors erreichbar ist.

Die Tastschalterbaugruppe 10 mit dem vorangehend beschriebenen Aufbau wird nach dem Zusammensetzen derselben in die im Handstückgehäuse 12 vorgesehene Tastschalteraufnahmeaussparung 24 eingesetzt. Dabei entspricht vorteilhafterweise die Geometrie bzw. Kontur einer Innenumfangsfläche 116 der Tastschalteraufnahmeaussparung 24 der Außenumfangskontur des Trägers 28. Hier ist vorteilhafterweise jeweils eine kreisrunde Kontur gewählt. Dieses hat fertigungstechnische Vorteile und vermeidet bei minimalem Umfang der Tastschalterbaugruppe das Entstehen ecken- oder kantenartiger Bereiche, welche gegen das Eindringen von Fluid nur schwer abzudichten sind.

In Zuordnung zur Tastschalteraufnahmeaussparung 24 ist wenigstens eine, vorzugsweise eine Mehrzahl von Haltestufen 118 vorgesehen, welche über die Innenumfangsfläche 116 nach innen hin vorstehen. Diese Halterstufen 118 oder eine beispielsweise ringartig umlaufende Haltestufe können einerseits einen Einschubanschlag für die gesamte Tastschalterbaugruppe 26 bilden, um somit beim Einsetzen derselben in das Handstückgehäuse 12 eine definierte Einbauposition vorzugeben. Andererseits kann mit derartigen Haltestufen 118 die Schalteranordnung 58 in definierter Positionierung bezüglich des ringartigen Trägers 28 gehalten werden. Dies bedeutet, dass keine zusätzlichen Fixiermaßnahmen für die Schalteranordnung 58 vorgesehen sein müssen, um diese im Trägerinnenraum 56 zu halten. Gleichwohl könnte in einem Vormontagezustand ein Herausfallen der Schalteranordnung 58 aus dem ringartigen Träger durch einen beim Einsetzen der Schalteranordnung 58 in den Trägerinnenraum 56 generierten Presssitz, beispielsweise der Positionierungsvorsprünge 90, 92 in den Positionierungsvorsprüngen 96, 98, verhindert werden.

Die Tastschalteranordnung 26 wird in der Tastschalteraufnahmeaussparung 24 durch das nach radial außen gegen die Innenumfangsfläche 116 pressende Dichtelement 38 am Handstückgehäuse 12 fixiert. Dabei stützt sich das Dichtelement 38 nach radial innen hin am Nutgrund der Nut 38 des Trägers 28 und nach radial außen an der der Außenumfangsfläche 40 mit geringem Abstand gegenüberliegenden Innenumfangsfläche 116 ab. Das Dichtelement 38 erfüllt somit nicht nur die Funktionalität des Abdichtens der Tastschalterbaugruppe 26 bezüglich des Handstückgehäuses 12, sondern erfüllt auch die Funktionalität der Fixierung der Tastschalterbaugruppe 26 am Handstückgehäuse 12. Alternativ oder zusätzlich zu der durch das Dichtelement 38 hervorgerufenen Fixierwirkung könnte diese Fixierung auch durch einen Presssitz erfolgen, der zwischen der Innenumfangsfläche 116 einerseits und der Außenumfangsfläche 40 des ringartigen Trägers 28 andererseits, insbesondere den in der Darstellung der Figur 3 über der Nut 36 und somit angrenzend an die Stirnseite 48 liegenden Abschnitt derselben, erzeugt wird. Dies vermeidet die Notwendigkeit, zusätzliche Fixierorgane, wie z. B. Schrauben oder dergleichen, einzusetzen, welche die Gefahr der Erzeugung von zur Durchführung von Reinigungsvorgängen nur schwer zugänglichen Hinterschneidungsbereichen oder Innenräumen mit sich bringen.

Mit der erfindungsgemäßen Ausgestaltung einer Tastschaltbaugruppe für ein Handstück zur Durchführung medizinischer Eingriffe bzw. eines mit einer derartigen Tastschaltbaugruppe ausgebildeten Handstücks wird bei einfachem Aufbau eine fluiddichte Anbindung der Tastschaltbaugruppe an ein Handstückgehäuse gewährleistet, ohne dass hierfür zusätzliche Befestigungsorgane erforderlich sind. Wesentlich hierfür ist der Einsatz einer radial wirksamen Dichtungsanordnung, welche nicht nur den fluiddichten Anschluss, sondern auch die Fixierung der Tastschalterbaugruppe am Handstückgehäuse gewährleistet. Es ist selbstverständlich, dass an einem Handstück mehrere derartige Tastschalterbaugruppen ggf. auch mit unterschiedlicher Anzahl an Schaltfunktionen vorgesehen sein können.

## Patentansprüche

1. Tastschalterbaugruppe für ein Handstück zur Durchführung medizinischer Eingriffe, umfassend:
- einen einen Trägerinnenraum (56) umgebenden ringartigen Träger (28),
- an dem Träger (28) ein zur Durchführung von Schaltvorgängen zu betätigendes Tasterelement (42), wobei das Tasterelement (42) an dem Träger (28) den Trägerinnenraum (56) fluiddicht abschließend festgelegt ist,
wobei an einer Außenumfangsseite (34) des ringartigen Trägers (28) eine den ringartigen Träger (28) umgebende Dichtungsanordnung (36, 38) vorgesehen ist, und wobei eine Schalteranordnung (58) mit wenigstens einem durch das Tasterelement (42) beaufschlagbaren Schalterelement (66, 68, 70) vorgesehen ist und eine im Trägerinnenraum (56) angeordnete und wenigstens ein Schalterelement (66, 68, 70) tragende Schalterplatte (62) umfasst, wobei das Tasterelement (42) in Zuordnung zu wenigstens einem Schalterelement (66, 68, 70) einen Beaufschlagungsbereich (82, 84, 86) aufweist, **dadurch gekennzeichnet, dass** wenigstens ein Beaufschlagungsbereich (82, 84, 86) einen an einer zum Trägerinnenraum (56) hin orientierten Innenseite (112) des Tasterelements (42) vorgesehenen zweiten Beaufschlagungsvorsprung (114) umfasst.

2. Tastschalterbaugruppe nach Anspruch 1,
**dadurch gekennzeichnet, dass** das Tasterelement (42) im Wesentlichen plattenartig ausgebildet ist und zur fluiddichten Festlegung am Träger (28) einen eine Stirnseite (48) des Trägers (28) wenigstens bereichsweise übergreifenden Befestigungsflansch (50) aufweist oder/und eine in den Trägerinnenraum (56) eingreifende und an einer Innenumfangsseite (30) des Trägers (28) anliegende Befestigungswandung (54) aufweist.

3. Tastschalterbaugruppe nach Anspruch 1 oder 2,
**dadurch gekennzeichnet, dass** das Tasterelement (42) an dem Träger (28) durch Materialschluss, vorzugsweise Anvulkanisieren, festgelegt ist.

4. Tastschalterbaugruppe nach einem der Ansprüche 1 bis 3,
**dadurch gekennzeichnet, dass** die Dichtungsanordnung (38) an der Außenumfangsseite (34) des Trägers (28) in wenigstens einer Dichtelementaufnahmenut (36) wenigstens ein O-ringartiges Dichtelement (38) umfasst.

5. Tastschalterbaugruppe nach einem der Ansprüche 1 bis 4,
**dadurch gekennzeichnet, dass** der Träger (28) eine im Wesentlichen runde, vorzugsweise kreisrunde, Außenumfangskontur aufweist.

6. Tastschalterbaugruppe nach einem der Ansprüche 1 bis 5,**dadurch gekennzeichnet, dass** am Tasterelement (42) oder/und am Träger (28) eine Positionierungsformation (88) vorgesehen ist und dass an der Schalterplatte (62) eine mit der Positionierungsformation (88) zum Vorgeben einer vorbestimmten Positionierung für die Schalterplatte (62) im Trägerinnenraum (56) zusammenwirkende Gegen-Positionierungsformation (94) vorgesehen ist.

7. Tastschalterbaugruppe nach Anspruch 6,
**dadurch gekennzeichnet, dass** eine Formation von Positionierungsformation (88) und Gegen-Positionierungsformation (94), vorzugsweise die Positionierungsformation (88), wenigstens einen Positioniervorsprung (90, 92) umfasst und die andere Formation von Positionierungsformation (88) und Gegen-Positionierungsformation (94), vorzugsweise die Gegen-Positionierungsformation (94), wenigstens eine wenigstens einen Positioniervorsprung (90, 92) aufnehmende Positionieraussparung (96, 98) umfasst.

8. Tastschalterbaugruppe nach einem der Ansprüche 1 bis 7,
**dadurch gekennzeichnet, dass** das Tasterelement (42) in Zuordnung zu jedem Schalterelement (66, 68, 70) einen Beaufschlagungsbereich (82, 84, 86) aufweist.

9. Tastschalterbaugruppe nach Anspruch 8,
**dadurch gekennzeichnet, dass** wenigstens ein Beaufschlagungsbereich (82, 84, 86) eine an einer vom Trägerinnenraum (56) weg orientierten Außenseite (108) des Tasterelements (42) vorgesehene erste Beaufschlagungsformation (110), vorzugsweise erster Beaufschlagungsvorsprung (110).

10. Tastschalterbaugruppe nach einem der Ansprüche 8 oder 9,
**dadurch gekennzeichnet, dass** in Zuordnung zu wenigstens einem Schalterelement (66, 68, 70), vorzugsweise in Zuordnung zu jedem Schalterelement (66, 68, 70), ein der Beaufschlagungswirkung des Tasterelements (42) entgegenwirkendes, vorzugsweise monostabiles Beaufschlagungswiderstandskraftelement (74, 76, 78) vorgesehen ist.

11. Tastschalterbaugruppe nach Anspruch 10,
**dadurch gekennzeichnet, dass** wenigstens ein Beaufschlagungswiderstandskraftelement (74, 76, 78) durch eine Fixierlage (80), vorzugsweise Klebefolie (80), bezüglich der Schalteranordnung (58) fixiert ist.

12. Handstück zur Durchführung medizinischer Eingriffe, umfassend wenigstens eine Tastschalterbaugruppe (26) nach einem der vorhergehenden Ansprüche, wobei in dem Handstück (10) in Zuordnung zu der wenigstens einen Tastschalterbaugruppe (26) eine diese aufnehmende Tastschalteraufnahmeaussparung (24) vorgesehen ist.

13. Handstück nach Anspruch 12,
**dadurch gekennzeichnet, dass** die Dichtungsanordnung (36, 38) an einer die Tastschalteraufnahmeaussparung (24) umgebenden Innenumfangsfläche (116) fluiddicht anliegt oder/und dass bei der Tastschalteraufnahmeaussparung (24) wenigstens eine Haltestufe (118) für die Tastschalterbaugruppe vorgesehen ist.

## Claims

1. Pushbutton switch assembly for a hand piece for performing medical procedures, comprising:
- a ring-like carrier (28) surrounding a carrier interior space (56),
- at the carrier (28), a pushbutton element (42) to be activated for carrying out a switch operation, wherein the pushbutton element (42) is attached to the carrier (28) sealing the carrier interior space (56) in a fluid-tight manner, wherein at an outer circumferential side (34) of the ring-like carrier (28) a seal arrangement (36, 38) is provided, surrounding the ring-like carrier (28), and wherein a switch arrangement (58) is provided with at least one switch element (66, 68, 70) to be activated by the pushbutton element (42) and comprises a switch plate (62) arranged in the carrier interior space (56) and carrying at least one switch element (66, 68, 70), wherein the pushbutton element (42) comprises an actuation area (82, 84, 86) associated to at least one switch element (66, 68, 70), **characterized in that** at least one actuation area (82, 84, 86) comprises a second actuation projection (114) at an inner side (112) of the pushbutton element (42) oriented towards the carrier interior space (56).

2. Pushbutton switch assembly according to claim 1,
**characterized in that** the pushbutton element (42) is adapted essentially platelike and comprises an attachment flange (50) partially engaging a front side (48) of the carrier (28) or/and an attachment wall (54) engaging the carrier interior space (56) and abutting at an inner circumferential side (30) of the carrier (28) for a fluid-tight attachment at the carrier (28).

3. Pushbutton switch assembly according to claim 1 or 2,
**characterized in that** the pushbutton element (42) is attached to the carrier (28) by material fit, preferably by vulcanization.

4. Pushbutton switch assembly according to one of claims 1 to 3,
**characterized in that** the sealing assembly (38) comprises at the outer circumferential side (34) of the carrier (28) in at least one sealing element receiving groove (36) at least one O-ring-like sealing element (38).

5. Pushbutton switch assembly according to one of claims 1 to 4,
**characterized in that** the carrier (28) has a substantially round, preferably circular outer circumferential contour.

6. Pushbutton switch assembly according to one of claims 1 to 5,
**characterized in that** a positioning structure (88) is provided at the pushbutton element (42) or/and at the carrier (28) and **in that** a counter positioning structure (94) cooperating with the positioning structure (88) for defining a predetermined positioning of the switch plate (62) in the carrier interior space (56) is provided at the switch plate (62).

7. Pushbutton switch assembly according to claim 6,
**characterized in that** one structure out of positioning structure (88) and counter positioning structure (94), preferably the positioning structure (88) comprises at least one positioning projection (90, 92) and the other structure out of positioning structure (88) and counter positioning structure (94), preferably the counter positioning structure (94), comprises at least one positioning recess (96, 98) receiving at least one positioning projection (90, 92).

8. Pushbutton switch assembly according to one of claims 1 to 7,
**characterized in that** the pushbutton element (42) comprises an actuation area (82, 84, 86) in association to each switch element (66, 68, 70).

9. Pushbutton switch assembly according to claim 8,
**characterized in that** at least one actuation area (82, 84, 86) comprises a first actuation structure (110), preferably a first actuation projection (110) provided at an outer side (108) of the pushbutton element (42) facing away from the carrier interior space (56).

10. Pushbutton switch assembly according to one of claims 8 or 9,
**characterized in that** a preferably monostable actuation resistance force element (74, 76, 78) counteracting the actuation effect of the pushbutton element (42) is provided in association to at least one switch element (66, 68, 70), preferably in association to each switch element (66, 68, 70).

11. Pushbutton switch assembly according to claim 10,
**characterized in that** at least one actuation resistance force element (74, 76, 78) is fixed in relation to the switch arrangement (58) by a fixation layer (80), preferably an adhesive foil (80).

12. Hand piece for performing medical procedures, comprising at least one pushbutton switch assembly (26) according to one of the preceding claims, wherein a pushbutton switch receiving recess (24) receiving the pushbutton switch assembly (26) is provided at the hand piece (10) in association to the at least one pushbutton switch assembly (26).

13. Hand piece according to claim 12,
**characterized in that** the sealing assembly (36, 38) abuts in a fluid-tight manner at an inner circumferential surface (116) surrounding the pushbutton switch receiving recess (24) or/and **in that** at least one retention element (118) for the pushbutton switch assembly is provided at the pushbutton switch receiving recess (24).

## Revendications

1. Ensemble de bloc de bouton-poussoir pour une pièce à main destinée à l'exécution d'opérations médicales, comprenant :
un support annulaire (28) entourant un espace intérieur de support (56), - au support (28) un élément de poussoir (42) à activer pour exécuter des opérations de commutation, l'élément de poussoir (42) étant attaché au support (28) en fermant l'espace intérieur de support (56) de manière étanche au fluide,
un arrangement de joint (36, 38) entourant le support annulaire (28) étant prévu à un côté circonférentiel extérieur (34) du support annulaire (28), et un arrangement de bouton (58) étant prévu avec au moins un élément de bouton (66, 68, 70) qui peut être activé par l'élément de poussoir (42) et comprenant une plaque de bouton (62) arrangé dans l'espace intérieur de support (56) qui supporte au moins un élément de bouton (66, 68, 70), l'élément de poussoir (42) comprenant une région d'actuation (82, 84, 86) associée à au moins un élément de bouton (66, 68, 70), **caractérisé en ce qu'**au moins une région d'actuation (82, 84, 86) comprend une deuxième projection d'actuation (114) prévue á un côté intérieur (112) orienté vers l'espace intérieur de support (56).

2. Ensemble de bloc de bouton-poussoir selon la revendication 1, **caractérisé en ce que** l'élément de poussoir (42) est adapté essentiellement sous forme de plaque et comprend pour la fixation étanche au fluide au support (28), une flasque de fixation (50) engageant au moins partiellement une face frontale (48) du support (28) ou/et une paroi de fixation (54) engageant l'espace intérieur de support (56) et adjacente à un côté circonférentiel intérieur (30) du support (28).

3. Ensemble de bloc de bouton-poussoir selon la revendication 1 ou 2,
**caractérisé en ce que** l'élément de poussoir (42) est fixé au support (28) par complémentarité de matériau, de préférence par une vulcanisation.

4. Ensemble de bloc de bouton-poussoir selon une des revendications 1 à 3,
**caractérisé en ce que** l'arrangement de joint (38) comprend à un côté circonférentiel extérieur (34) du support (28) dans au moins une rainure de réception d'élément de joint (36) au moins un élément de joint en anneau O (38).

5. Ensemble de bloc de bouton-poussoir selon une des revendications 1 à 4,
**caractérisé en ce que** le support (28) comprend un contour circonférentiel extérieur essentiellement rond, de préférence circulaire.

6. Ensemble de bloc de bouton-poussoir selon une des revendications 1 à 5,
**caractérisé en ce qu'**une structure de positionnement (88) est prévue à l'élément de poussoir (42) ou/et au support (28) et **en ce qu'**une structure de positionnement opposée (94) coopérant avec la structure de positionnement (88) pour définir un positionnement prédéterminé pour la plaque de bouton (62) est prévue dans l'espace intérieur de support (56).

7. Ensemble de bloc de bouton-poussoir selon la revendication 6,
**caractérisé en ce qu'**une structure parmi structure de positionnement (88) et structure de positionnement opposée (94), de préférence la structure de positionnement (88), comprend au moins une projection de positionnement (90, 92) et que l'autre structure parmi structure de positionnement (88) et structure de positionnement opposée (94), de préférence la structure de positionnement opposée (94), comprend au moins une encoche de positionnement (96, 98) recevant au moins une projection de positionnement (90, 92).

8. Ensemble de bloc de bouton-poussoir selon une des revendications 1 à 7,
**caractérisé en ce que** l'élément de poussoir (42) comprend une région d'actuation (82, 84, 86) en association à chaque élément de bouton (66, 68, 70).

9. Ensemble de bloc de bouton-poussoir selon la revendication 8,
**caractérisé en ce qu'**au moins une région d'actuation (82, 84, 86) comprend une première structure d'actuation (110) prévue à un côté extérieur (108) de l'élément de poussoir (42) orienté côté opposé à l'espace intérieur de support (56), de préférence une première projection d'actuation (110).

10. Ensemble de bloc de bouton-poussoir selon une des revendications 8 ou 9,
**caractérisé en ce que** un élément de force de résistance d'actuation (74, 76, 78), de préférence monostable, agissant contre l'effet d'actuation de l'élément de poussoir (42) est prévu en association à au moins un élément de bouton (66, 68, 70), de préférence en association à chaque élément de bouton (66, 68, 70).

11. Ensemble de bloc de bouton-poussoir selon la revendication 10,
**caractérisé en ce qu'**au moins un élément de force de résistance d'actuation (74, 76, 78) est fixé par rapport à par une couche de fixation (80), de préférence par un film adhésif (80) par rapport à l'arrangement de bouton (58).

12. Pièce à main destinée à l'exécution d'opérations médicales comprenant au moins un ensemble de bouton-poussoir (26) selon une des revendications précédentes, une encoche de réception de bouton-poussoir (24) étant prévue dans la pièce à main (10) en association audit au moins un ensemble de bouton-poussoir (26).

13. Pièce à main selon la revendication 12,
**caractérisée en ce que** l'arrangement de joint (36, 38) est adjacent de manière étanche au fluide à une surface circonférentielle intérieure (116) entourant l'encoche de réception de bouton-poussoir (24) ou/et **en ce que** dans l'encoche de réception de bouton-poussoir (24) au moins un degré de maintien (118) est prévu pour l'ensemble de bloc de bouton-poussoir.
